# EUROPÄISCHE PATENTSCHRIFT

(11) **EP 3 041 916 B1**
(45) Veröffentlichungstag und Bekanntmachung des Hinweises auf die Patenterteilung: **25.10.2017**
(21) Anmeldenummer: 14758568.1
(22) Anmeldetag: 03.09.2014
(51) Int. Cl.: C10G 45/58, C10G 65/04, C10G 69/06, C10G 9/06, C10G 9/36, C10G 70/04, C07C 5/333, C07C 11/167, C07C 5/13, C07C 11/08, C07C 7/148

(54) **VERFAHREN ZUR ERZEUGUNG VON KOHLENWASSERSTOFFPRODUKTEN**
METHOD FOR PRODUCING HYDROCARBON PRODUCTS
PROCÉDÉ DE PRODUCTION D'HYDROCARBURES

(30) Priorität: 05.09.2013 DE 102013014866; 25.09.2013 EP 13004662
(43) Veröffentlichungstag der Anmeldung: 13.07.2016
(73) Patentinhaber: Linde Aktiengesellschaft, 80331 München (DE)
(72) Erfinder: WALTER, Stefanie, 82418 Seehausen (DE); FRITZ, Helmut, 81375 München (DE); SCHMIDT, Gunther, 82041 Deisenhofen (DE)
(86) Internationale Anmeldenummer: PCT/EP2014/068708
(87) Internationale Veröffentlichungsnummer: WO 2015/032804

(56) Entgegenhaltungen:
- EP-A1- 2 062 865
- US-A- 3 922 216
- US-A- 4 091 046
- US-A- 5 523 502
- US-A1- 2011 112 345

## Beschreibung

Die Erfindung betrifft ein Verfahren zur Erzeugung von Kohlenwasserstoffprodukten.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben.

Die EP 2 062 865 A1 offenbart ein Verfahren zur selektiven Herstellung von Ethylen, Propylen und Isopren aus leichten Kohlenwasserstoffen, das umfasst, eine Butanfraktion in eine an iso-Butan angereicherte Fraktion und eine an n-Butan angereicherte Fraktion zu trennen und die an n-Butan angereicherte Fraktion, gegebenenfalls zusammen mit weiteren Fraktionen, durch Dampfspalten zu bearbeiten.

Aus der US 4,091,046 A ist ein Verfahren zur Herstellung von iso-Amylen aus iso-Butan bekannt. Die US 2011/0112345 A1 offenbart ein Verfahren zur Herstellung eines Olefinprodukts, das Ethylen und/oder Propylen umfasst, bei dem ein parafinischer Einsatz durch Dampfspalten bearbeitet wird. Verfahren zur Behandlung von Kohlenwasserstoffströmen sind auch aus der US 5,523,502 A, der US 4,324,938 A und der FR 2 436 176 A1 bekannt.

In neueren Verfahren und Vorrichtungen zum Dampfspalten kommen zunehmend milde Spaltbedingungen zum Einsatz (siehe unten), weil sich bei diesen insbesondere sogenannte Wertprodukte, beispielsweise Propylen und Butadien, mit verbesserter Ausbeute gewinnen lassen, wie unten erläutert. Gleichzeitig verringert sich bei milden Spaltbedingungen jedoch auch die Umsetzung des Ofeneinsatzes, so dass sich in diesem enthaltene Verbindungen in vergleichsweise großer Menge im Spaltgas wiederfinden und zu einer "Verdünnung" der Wertprodukte führen.

Aufgabe vorliegender Erfindung ist es, hier Abhilfe zu schaffen und unter Beibehaltung der Vorteile der milden Spaltbedingungen deren Nachteile zu vermeiden. Insbesondere soll durch eine Reduzierung des erwähnten Verdünnungseffekts die Konzentration und Menge der Wertprodukte, insbesondere von 1,3-Butadien, erhöht werden.

### Offenbarung der Erfindung

Diese Aufgabe wird durch ein Verfahren zur Erzeugung von Kohlenwasserstoffprodukten mit den Merkmalen des Anspruchs 1 gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Dampfspaltverfahren werden im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, gegebenenfalls aber auch unter einheitlichen Spaltbedingungen betriebene Rohrreaktoren insgesamt, werden nachfolgend jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine Anlage zum Dampfspalten kann einen oder mehrere derartiger Spaltöfen aufweisen.

Mit dem Begriff "Ofeneinsatz" werden hier ein oder mehrere flüssige und/oder gasförmige Ströme bezeichnet, die einem oder mehreren Spaltöfen zugeführt werden. Auch durch ein entsprechendes Dampfspaltverfahren erhaltene Ströme, wie unten erläutert, können in einen oder mehrere Spaltöfen zurückgeführt und erneut als Ofeneinsatz verwendet werden. Als Ofeneinsatz eignet sich eine Vielzahl von Kohlenwasserstoffen und Kohlenwasserstoffgemischen von Ethan bis Gasöl bis zu einem Siedepunkt von typischerweise 600 °C.

Ein Ofeneinsatz kann aus einem sogenannten "Frischeinsatz" bestehen, also aus einem Einsatz, der anlagenextern bereitgestellt und beispielsweise aus einer oder mehreren Erdölfraktionen, Erdgaskomponenten mit zwei bis vier Kohlenstoffatomen und/oder Erdgaskondensaten gewonnen wird. Ein Ofeneinsatz kann auch aus einem oder mehreren sogenannten "Recycleströmen" bestehen, also Strömen, die in der Anlage selbst erzeugt und in einen entsprechenden Spaltofen zurückgeführt werden. Ein Ofeneinsatz kann auch aus einem Gemisch eines oder mehrerer Frischeinsätze mit einem oder mehreren Recycleströmen bestehen.

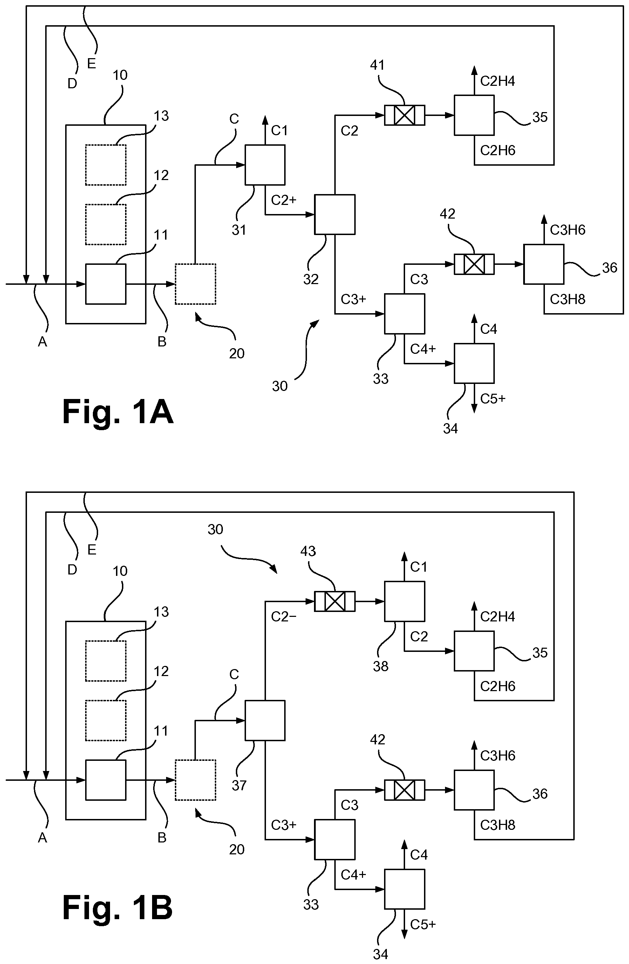

Der Ofeneinsatz wird im jeweiligen Spaltofen zumindest teilweise umgesetzt und verlässt den Spaltofen als sogenanntes "Rohgas", das, wie unten unter Bezugnahme auf die Figuren 1A und 1B erläutert, einer Reihe von Nachbehandlungsschritten unterworfen werden kann. Derartige Nachbehandlungsschritte umfassen zunächst eine Aufbereitung des Rohgases, beispielsweise durch Quenchen, Kühlen und Trocknen, wodurch ein sogenanntes "Spaltgas" erhalten wird. Bisweilen wird auch bereits das Rohgas als Spaltgas bezeichnet.

Gängige Verfahren umfassen insbesondere die Trennung des Spaltgases in eine Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für eine "C5-Fraktion" und die höheren Fraktionen. Mehrere solcher Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Der Begriff "überwiegend" bezeichnet einen Gehalt von wenigstens 50%, 60%, 70%, 80% oder 90% oder entspricht dem Begriff "reich". Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ein von einem entsprechenden Ausgangsstrom "abgeleiteter" Strom kann beispielsweise durch Abzweigen oder Abtrennen von dem Ausgangsstrom oder durch Vereinigen mit wenigstens einem weiteren Strom gebildet werden.

Die erwähnten "Spaltbedingungen" in einem Spaltofen umfassen unter anderem den Partialdruck des Ofeneinsatzes, der durch die Zugabe unterschiedlicher Dampfmengen und den im Spaltofen eingestellten Druck beeinflusst werden kann, die Verweildauer im Spaltofen sowie die in diesem verwendeten Temperaturen und Temperaturprofile. Auch die Ofengeometrie und Ofengestaltung spielt eine Rolle. Zur Herstellung von Ethylen wird ein Spaltofen beispielsweise bei einer Ofeneintrittstemperatur von 500 bis 680 °C und bei einer Ofenaustrittstemperatur von 775 bis 875 °C betrieben. Hierbei ist die "Ofeneintrittstemperatur" die Temperatur eines Gasstroms am Beginn eines Reaktionsrohrs und die "Ofenaustrittstemperatur" die Temperatur eines Gasstroms am Ende eines Reaktionsrohrs. Typischerweise handelt es sich bei letzterer um die maximale Temperatur, auf die der entsprechende Gasstrom aufgeheizt wird. Dem Ofeneinsatz wird dabei bei einem, ebenfalls am Ofenausgang gemessenen, Druck von 165 bis 225 kPa Dampf in einem Verhältnis von typischerweise 0,25 bis 0,85 kg/kg beigemischt. Die spezifisch verwendeten Werte sind vom jeweils verwendeten Ofeneinsatz und den erwünschten Spaltprodukten abhängig.

Da die genannten Werte einander zumindest teilweise wechselseitig beeinflussen, hat sich zur Charakterisierung der Spaltbedingungen der Begriff der "Spaltschärfe" (engl. Cracking Severity) durchgesetzt. Für flüssige Ofeneinsätze kann die Spaltschärfe über das Verhältnis von Propylen zu Ethylen (P/E) oder als das Verhältnis von Methan zu Propylen (M/P) im Spaltgas auf Gewichtsbasis (kg/kg) beschrieben werden. Das P/E- und das M/P-Verhältnis sind direkt von der Temperatur abhängig, können aber im Gegensatz zur realen Temperatur im oder am Ausgang eines Spaltofens sehr viel genauer gemessen und beispielsweise als Regelgröße in einem entsprechenden Regelungsverfahren verwendet werden. Das P/E-Verhältnis eignet sich aber nur bedingt zur Charakterisierung der Spaltschärfe bei gasförmigen Ofeneinsätzen bzw. Verbindungen mit zwei bis vier Kohlenstoffatomen.

Für gasförmige Ofeneinsätze kann die Umsetzung bzw. Konversion einer jeweils betrachteten Komponente des Ofeneinsatzes als Maß der Spaltschärfe angegeben werden. Der Begriff Umsetzung bzw. Konversion (engl. Conversion) wird hier wie in der Fachwelt üblich verwenden (siehe beispielsweise den erwähnten Artikel "Ethylene" in Ullmann's Encyclopedia of Industrial Chemistry). Insbesondere für die im vorliegenden Fall eingesetzten C4-Fraktionen bzw. C4-Teilströme ist eine Beschreibung der Spaltschärfe über die Umsetzung von Schlüsselkomponenten wie n-Butan und iso-Butan gut geeignet.

Die Spaltschärfen bzw. Spaltbedingungen sind "scharf", wenn n-Butan in einer entsprechenden Fraktion zu mehr als 92% umgesetzt wird. Bei noch schärferen Spaltbedingungen wird n-Butan ggf. auch zu mehr als 93%, 94% oder 95% umgesetzt. Eine Umsetzung von n-Butan zu 100% erfolgt typischerweise nicht. Die Obergrenze der "scharfen" Spaltschärfen bzw. Spaltbedingungen liegt daher beispielsweise bei 99%, 98%, 97% oder 96% Umsetzung von n-Butan. Die Spaltschärfen bzw. Spaltbedingungen sind hingegen "mild", wenn n-Butan zu weniger als 92% umgesetzt wird. Bei weniger als 91%, weniger als 90%, weniger als 89%, weniger als 88% oder weniger als 87% Umsetzung von n-Butan liegen zunehmend mildere Spaltschärfen bzw. Spaltbedingungen vor. Bei weniger als 86% Umsetzung von n-Butan werden die Spaltschärfen bzw. Spaltbedingungen hier als "sehr mild" bezeichnet. Sehr milde Spaltschärfen bzw. Spaltbedingungen umfassen auch beispielsweise eine Umsetzung von n-Butan zu weniger als 85%, 84%, 83%, 82%, 81%, 80%, 79%, 78%, 77%, 76%, 75%, 70% oder 65% und zu mehr als 50% oder 60%.

Die Spaltschärfen bzw. die Spaltbedingungen sind auch dann "scharf", wenn iso-Butan in einer entsprechenden Fraktion zu mehr als 91% umgesetzt wird. Bei noch schärferen Spaltbedingungen wird iso-Butan ggf. auch zu mehr als 92%, 93% oder 94% umgesetzt. Auch eine Umsetzung von iso-Butan zu 100% erfolgt typischerweise nicht. Die Obergrenze der "scharfen" Spaltschärfen bzw. Spaltbedingungen liegt daher beispielsweise bei 99%, 98%, 97% oder 96% Umsetzung von iso-Butan. Die Spaltschärfen bzw. die Spaltbedingungen sind hingegen "mild", wenn iso-Butan zu weniger als 91% umgesetzt wird. Bei weniger als 90%, weniger als 89%, weniger als 88%, weniger als 87% oder weniger als 86% Umsetzung von iso-Butan liegen zunehmend mildere Spaltschärfen bzw. Spaltbedingungen vor. Bei weniger als 83% Umsetzung von iso-Butan werden die Spaltschärfen bzw. Spaltbedingungen hier als "sehr mild" bezeichnet. Sehr milde Spaltschärfen bzw. Spaltbedingungen umfassen auch beispielsweise eine Umsetzung von iso-Butan zu weniger als 82%, 81%, 80%, 79%, 78%, 77%, 76%, 75% oder 70% und zu mehr als 45% oder 50%.

Die genannten Spaltschärfen bzw. Spaltbedingungen hängen insbesondere mit der oben erläuterten Ofenaustrittstemperatur am Ende des oder der jeweils verwendeten Reaktionsrohre bzw. Spaltöfen zusammen. Je höher diese ist, desto "schärfer", je niedriger, desto "milder" sind die Spaltschärfen bzw. Spaltbedingungen.

Ferner versteht sich, dass die Umsetzung anderer Komponenten nicht identisch mit jener des n- und des iso-Butans sein muss. Werden beispielsweise 1- und 2-Buten zusammen mit n-Butan gespalten, so werden diese typischerweise in größerem Umfang umgesetzt als das n-Butan. Umgekehrt wird iso-Buten in geringerem Umfang als iso-Butan umgesetzt, wenn es zusammen mit diesem gespalten wird. Mit einer prozentualen Umsetzung einer Schlüsselkomponente, hier n-Butan bzw. iso-Butan, sind also jeweils eine Ofenaustrittstemperatur und die jeweiligen prozentualen Umsetzungen der anderen Komponenten im Einsatz verknüpft. Diese Ofenaustrittstemperatur ist wiederum unter anderem vom Spaltofen abhängig. Der Versatz zwischen den jeweiligen prozentualen Umsetzungen ist von einer Reihe von weiteren Faktoren abhängig.

### Vorteile der Erfindung

Die vorliegende Erfindung geht von einem Verfahren zur Erzeugung von Kohlenwasserstoffprodukten aus, bei dem ein Kohlenwasserstoffstrom bereitgestellt wird, der überwiegend verzweigte und unverzweigte Kohlenwasserstoffe mit vier Kohlenstoffatomen aufweist (nachfolgend auch als C4-Fraktion oder C4-Strom bezeichnet, Kurzbezeichnung C4). Insoweit entspricht das erfindungsgemäße Verfahren beispielsweise bekannten Verfahren zur Erzeugung von Kohlenwasserstoffprodukten durch Dampfspalten, bei denen aus einem Spaltgas, das ggf. entsprechend aufbereitet wurde, eine derartige C4-Fraktion abgetrennt wird. Dies kann in bekannten Anlagen in einem sogenannten Debutanizer erfolgen (der jedoch auch alle anderen Kohlenwasserstoffe mit vier Kohlenstoffatomen aus einem entsprechenden Kohlenwasserstoffstrom abtrennt). Details hierzu sind in dem erwähnten Artikel "Ethylene" in Ullmann's Encyclopedia of Industrial Chemistry gezeigt und unter Bezugnahme auf die Figuren 1A und 1B veranschaulicht.

Die Erfindung ist jedoch nicht auf die Verwendung von C4-Strömen beschränkt, die durch Dampfspalten und nachgeordnete Verfahrensschritte bereitgestellt werden, sondern eignet sich in gleicher Weise auch für C4-Ströme, die zumindest teilweise mittels anderer Verfahren, beispielsweise mittels Raffinerieprozessen, erzeugt werden. Beispielsweise kann die Erfindung mit C4-Strömen verwendet werden, die zuvor nicht dampfgespalten wurden und erst anschließend einem entsprechenden Dampfspaltprozess zugeführt werden. Hierbei kann es sich beispielsweise um Erdölfraktionen, Erdgaskomponenten mit zwei bis vier Kohlenstoffatomen, Erdgaskondensate und dergleichen handeln.

Die Erfindung sieht nun vor, aus dem erwähnten Kohlenwasserstoffstrom, der überwiegend verzweigte und unverzweigte Kohlenwasserstoffe mit vier Kohlenstoffatomen aufweist, einen ersten und einen zweiten Teilstrom zu gewinnen, wobei der erste Teilstrom überwiegend verzweigte Kohlenwasserstoffe mit vier Kohlenstoffatomen und der zweite Teilstrom überwiegend unverzweigte Kohlenwasserstoffe mit vier Kohlenstoffatomen aufweist. Der erste Teilstrom wird nachfolgend auch als iso-C4-Fraktion oder iso-C4-Strom (Kurzbezeichnung iso-C4), der zweite Teilstrom auch als n-C4-Fraktion oder n-C4-Strom (n-C4) bezeichnet.

Die Erfindung sieht ferner vor, zumindest einen Teil des ersten Teilstroms oder eines hiervon abgeleiteten Stroms bei einer ersten, höheren Spaltschärfe und zumindest einen Teil des zweiten Teilstroms oder eines hiervon abgeleiteten Stroms bei einer zweiten, geringeren Spaltschärfe zu spalten, wobei die erste, höhere Spaltschärfe in einer Umsetzung von iso-Butan in dem ersten Teilstrom von mehr als 91% und bis zu 99% und die zweite, geringere Spaltschärfe in einer Umsetzung von n-Butan in dem zweiten Teilstrom von weniger als 92% und mehr als 50% resultiert. Das "Spalten" umfasst dabei, den entsprechenden Teilstrom (oder einen entsprechenden Anteil) alleine oder zusammen mit weiteren Strömen, ggf. auch nach voriger Vereinigung zu einem Sammelstrom, in einen Spaltofen gemäß der eingangs getroffenen Definition einzuspeisen und dem Spaltofen ein Spaltgas zu entnehmen.

Die zur Charakterisierung der Spaltschärfen verwendeten Begriffe "höher" und "geringer" beziehen sich aufeinander. Mit anderen Worten wird zumindest ein Teil des ersten Teilstroms bei einer ersten Spaltschärfe und zumindest ein Teil des zweiten Teilstroms bei einer zweiten Spaltschärfe gespalten, wobei die erste Spaltschärfe höher als die zweite und die zweite geringer als die erste ist.

Die Dampfspaltung von C4-Fraktionen unterschiedlicher Herkunft ist Stand der Technik. Eine zuverlässige Vorhersage des Spaltergebnisses ist mit den vorhandenen Werkzeugen möglich. In der Regel liegen diese als Gemische aus verzweigten und unverzweigten C4-Verbindungen vor. In den zuvor erwähnten Frischeinsätzen umfassen diese überwiegend paraffinische Verbindungen, in Recycleströmen von Dampfspaltprozessen oder in Produkten anderer Verarbeitungsverfahren (z.B. aus Raffinerien) überwiegend olefinische Verbindungen.

Insbesondere bei Spaltung von Naphthas bei milden Spaltbedingungen oder einem hohen Anteil an C4-Frischeinsätzen ist auch der Anteil der aus dem entsprechenden Spaltgas erhaltenen C4-Fraktion groß und insbesondere relativ schwachkonzentriert an 1,3-Butadien und ggf. anderen Wertprodukten, die aus der C4-Fraktion extrahiert werden sollen. Als Folge ist die Gewinnung von 1,3-Butadien unwirtschaftlich.

Insbesondere beim Dampfspalten von Kohlenwasserstoffen bei ungewöhnlich milden Spaltbedingungen kommt es also, wie erwähnt, zu einer deutlichen Mengenerhöhung einiger Produktfraktionen und einer damit verbundenen Reduktion der Konzentration enthaltener Wertprodukte (Verdünnungseffekt). Die Gewinnung der Wertprodukte wird dadurch erschwert bzw. kostspieliger.

Der Erfindung liegt die Erkenntnis zugrunde, dass verzweigte C4-Verbindungen im Spaltofen strukturbedingt in geringem Umfang zur Bildung von 1,3-Butadien beitragen. Eine relativ hohe Methanbildung aus derartigen Verbindungen ist unvermeidbar, insbesondere dann, wenn die verzweigten C4-Verbindungen bis zur vollständigen Umsetzung recycelt werden. Werden also C4-Gemische unter milden oder gar sehr milden Bedingungen gespalten, resultieren daraus C4-Fraktionen relativ hoher Mengenströme bei gleichzeitig niedriger Konzentration an 1,3-Butadien.

Diesem Effekt wird erfindungsgemäß begegnet, indem die C4-Fraktion vor der Dampfspaltung, beispielsweise destillativ, in iso- und n-C4-Teilströme (den erwähnten ersten und zweiten Teilstrom) aufgetrennt und unter unterschiedlichen Bedingungen gespalten werden. Der (erste) iso-C4-Teilstrom wird dabei schärfer als der (zweite) n-C4-Teilstrom gespalten, wobei der (erste) iso-C4-Teilstrom insbesondere sehr scharf, der (zweite) n-C4-Teilstrom insbesondere besonders mild gespalten wird.

Durch die Erfindung werden unter Beibehaltung ihrer Vorteile die Nachteile der milden Spaltung vermindert bzw. vollständig vermieden, d.h. die Menge der C4-Fraktion wird verringert und dadurch die Konzentration des Ziel produkts, hier insbesondere 1,3-Butadien erhöht. Der spezifische Extraktionsaufwand wird verringert.

Der Kern der Erfindung besteht also in der Minimierung der C4-Fraktion insgesamt durch gezielte Erhöhung der strukturellen Umsetzung von iso-C4-Verbindungen durch scharfe Spaltbedingungen nach einer vorherigen Abtrennung von n-C4-Verbindungen. Dadurch wird die selektive Anwendung milder, insbesondere sehr milder Spaltbedingungen auf die n-C4-Verbindungen ermöglicht, so dass die durch diese erzielbaren hohen Selektivitäten, z.B. in Richtung von 1,3-Butadien, erreicht werden.

Besondere Vorteile ergeben sich, wenn der erste Teilstrom, also jener, der überwiegend verzweigte Kohlenwasserstoffe mit vier Kohlenstoffatomen aufweist, vor dem Dampfspalten bei der ersten, höheren Spaltschärfe zumindest teilweise einem Hydrierverfahren unterzogen wird. Hierbei wird das enthaltene iso-Buten (olefinisch) zumindest teilweise zu iso-Butan (paraffinisch) umgesetzt. Im Rahmen der vorliegenden Erfindung hat sich herausgestellt, dass sich das iso-Butan im nachgeschalteten Spaltverfahren, d.h. bei der ersten, höheren Spaltschärfe, leichter bzw. zu besser verwertbaren Produkten umsetzen lässt. Dies ermöglicht eine weitere Verringerung der Menge der C4-Fraktion und dadurch eine Konzentration der Zielprodukte, wie oben erwähnt.

Zur Hydrierung von Olefinen oder olefinhaltigen Kohlenwasserstoffgemischen sind aus dem Stand der Technik zahlreiche katalytische Verfahren bekannt, die auch im Rahmen der vorliegenden Erfindung zum Einsatz kommen können. Hydrierkatalysatoren besitzen als hydrieraktive Komponente eines oder mehrere Elemente der 6., 7. oder 8. Nebengruppe des Periodensystems in elementarer oder gebundener Form. Sie können mit unterschiedlichen Zusätzen dotiert sein, um bestimmte Katalysatoreigenschaften, beispielsweise Lebensdauer, Resistenz gegen bestimmte Katalysatorgifte, Selektivität oder Regenerierbarkeit zu beeinflussen. Die Hydrierkatalysatoren enthalten die hydrieraktive Komponente vielfach auf Trägern, beispielsweise Mordeniten, Zeolithen, Al₂O₃-Modifikationen, SiO₂-Modifikationen und anderen. Zur weitgehenden Hydrierung der Olefine werden im Allgemeinen Reaktionstemperaturen von 150 bis 250 °C angewandt.

Die zuvor erläuterten Verfahrensvarianten können umfassen, den erwähnten Kohlenwasserstoffstrom, aus dem der erste und der zweite Teilstrom gewonnen wird, zumindest teilweise aus wenigstens einem Spaltgasstrom zu bilden, der beim erfindungsgemäßen Dampfspalten des ersten und zweiten Teilstroms oder entsprechender Anteile hiervon zusammen mit Frischeinsatz erzeugt wird.

Der Kohlenwasserstoffstrom kann jedoch auch zumindest teilweise aus einem Spaltgas, das durch Dampfspalten eines Frischeinsatzes erhalten wird, oder aus einem ungespaltenen Frischeinsatz gebildet werden. Diese Varianten ermöglichen eine sehr flexible Einstellung der jeweils erwünschten Gehalte des Kohlenwasserstoffstroms hinsichtlich der einzelnen C4-Verbindungen.

Erfindungsgemäß resultiert die erste, höhere Spaltschärfe, die für die iso-C4-Verbindungen verwendet wird, in einer Umsetzung des enthaltenen iso-Butans zu mindestens 91% oder mehr, wie oben zum Begriff der Spaltschärfe erläutert. Für die iso-C4-Verbindungen werden also scharfe oder sehr scharfe Spaltschärfen bzw. Spaltbedingungen eingesetzt. Diese können auch beispielsweise mehr als 92%, 93% oder 94% und bis zu 99% Umsetzung von iso-Butan entsprechen. Die zweite, geringere Spaltschärfe, die für die n-C4-Verbindungen verwendet wird, resultiert hingegen vorteilhafterweise in einer Umsetzung des enthaltenen n-Butans zu höchstens 92% oder weniger, wie ebenfalls oben erläutert. Für die n-C4-Verbindungen werden also milde oder sehr milde Spaltschärfen bzw. Spaltbedingungen eingesetzt. Diese können beispielsweise auch weniger als 90%, 88%, 86%, 84%, 82%, 80%, 78%, 76%, 74%, 72%, 70% oder 65%, jedoch beispielsweise mehr als 50% oder 60% Umsetzung von n-Butan entsprechen. Bei zunehmend milden Spaltbedingungen entstehen im Verhältnis zum Frischeinsatz mehr erwünschte Produkte wie Butadien und Propylen, so dass sich die Ausbeute erhöht und das Produktspektrum verbessert.

Die Spaltschärfen unterscheiden sich dabei, normiert auf einen gemeinsamen zugrundegelegten Wert, beispielsweise eine Umsetzung von n-Butan oder iso-Butan oder beidem, um 1 bis 20%, insbesondere um 2 bis 20%.

Entsprechende Verfahren umfassen vorteilhafterweise die Verwendung einer Dampfmenge von 0,4 kg/kg, insbesondere von 0,2 bis 0,7 kg/kg, beispielsweise von 0,3 bis 0,5 kg/kg bei der ersten, höheren Spaltschärfe und die Verwendung einer Dampfmenge von 0,4 kg/kg, insbesondere von 0,2 bis 0,7 kg/kg, beispielsweise von 0,3 bis 0,5 kg/kg bei der zweiten, geringeren Spaltschärfe. Die jeweils verwendeten Werte können gleich oder unterschiedlich sein und insbesondere auch an weitere gespaltene Einsätze angepasst werden.

Das erfindungsgemäße Verfahren erweist sich als besonders vorteilhaft, wenn das Dampfspalten bei der ersten, höheren Spaltschärfe und/oder das Dampfspalten bei der zweiten, geringeren Spaltschärfe jeweils in wenigstens einem Spaltofen durchgeführt wird, dem zumindest ein weiterer Ofeneinsatz zugeführt wird. Beispielsweise kann ein für einen entsprechenden Durchsatz ausgelegter Spaltofen verwendet werden, der bei der zweiten, geringeren Spaltschärfe betrieben wird und in dem neben dem zweiten Teilstrom mit den n-C4-Verbindungen auch ein "regulärer" Frischeinsatz mild gespalten wird. Die iso-C4-Verbindungen können alleine in einem Spaltofen gespalten werden. In bestimmten Fällen, beispielsweise wenn aus Kostengründen gleichartige Spaltöfen eingesetzt werden, kann es jedoch sinnvoller sein, die iso-C4-Verbindungen zusammen mit einem Frischeinsatz scharf zu spalten.

Wie erwähnt, hat die vorliegende Erfindung insbesondere den Zweck, ein Verfahren zu verbessern, bei dem 1,3-Butadien aus dem Kohlenwasserstoffstrom abgetrennt wird. Hierbei eignen sich sämtliche bekannten Verfahren zur Extraktion von 1,3-Butadien.

Weitere Vorteile können sich ergeben, wenn in dem Kohlenwasserstoffstrom enthaltenes Isobuten nach der Abtrennung des 1,3-Butadiens zumindest teilweise zu einem tert-Butylether umgesetzt und dieser ebenfalls extrahiert wird. Die Herstellung von Methyl-tert-butylether (2-Methoxy-2-methylpropan, MTBE) ist grundsätzlich bekannt. MTBE wird großtechnisch säurekatalytisiert aus Isobuten und Methanol hergestellt, das dem Kohlenwasserstoffstrom zugegeben wird. MTBE wird hauptsächlich als Klopfschutzmittel verwendet, findet aber auch zunehmend Verwendung als Lösungsmittel sowie Extraktionsmittel in der organischen Chemie. Ethanol ergibt Ethyl-tert-butylether. Auch andere Alkohole können eingesetzt werden.

Weiter vorteilhaft ist ein Verfahren, bei dem in dem Kohlenwasserstoffstrom enthaltenes 1-Buten nach der zumindest teilweisen Umsetzung des Isobutens zumindest teilweise zu 2-Buten hydroisomerisiert wird. Das erhaltene 2-Buten kann bei der anschließenden milden Spaltung besonders selektiv zum gewünschten Butadien umgesetzt werden. Auch entsprechende Hydroisomerisierungsverfahren sind bekannt. Beispielsweise kann hierdurch auch eine Entfernung von verbliebenen Spuren von Butadien erfolgen.

Es kann auch vorteilhaft sein, zu dem C4-Kohlenwasserstoffstrom, der überwiegend verzweigte und unverzweigte Kohlenwasserstoffe mit jeweils vier Kohlenstoffatomen aufweist, vor oder nach der Hydroisomerisierung oder einem anderen Prozess, wenigstens einen weiteren Strom, insbesondere einen Butine (C4-Acetylene) und/oder Kohlenwasserstoffe mit fünf Kohlenstoffatomen enthaltenden Strom zuzuspeisen. Beispielsweise können hierbei bei einer Butadienextraktion koextrahierte C5-Verbindungen verwendet und einer sinnvollen Nutzung zugeführt werden.

Die Erfindung wird nachfolgend unter Bezugnahme auf die beigefügten Figuren gegenüber dem Stand der Technik erläutert.

### Kurze Beschreibung der Zeichnungen

Figur 1A veranschaulicht schematisch den Ablauf eines Verfahrens zur Erzeugung von Kohlenwasserstoffen gemäß dem Stand der Technik.
Figur 1B veranschaulicht schematisch den Ablauf eines Verfahrens zur Erzeugung von Kohlenwasserstoffen gemäß dem Stand der Technik.
Figur 2 veranschaulicht schematisch den Ablauf eines Verfahrens zur Erzeugung von Kohlenwasserstoffen gemäß einer Ausführungsform der Erfindung.

In den Figuren sind einander entsprechende Elemente mit identischen Bezugszeichen versehen und werden der Übersichtlichkeit halber nicht wiederholt erläutert.

### Ausführliche Beschreibung der Zeichnungen

In Figur 1A ist der Ablauf eines Verfahrens zur Erzeugung von Kohlenwasserstoffen gemäß dem Stand der Technik in Form eines schematischen Ablaufplans dargestellt. Kern des Verfahrens ist hier ein Dampfspaltprozess 10, der unter Verwendung eines oder mehrerer Spaltöfen 11 bis 13 durchgeführt werden kann. Nachfolgend wird nur der Betrieb des Spaltofens 11 erläutert, die weiteren Spaltöfen 12 und 13 können in entsprechender Weise arbeiten.

Der Spaltofen 11 wird mit einem Strom A als Ofeneinsatz beschickt, bei dem es sich zumindest teilweise um einen sogenannten Frischeinsatz handeln kann, der aus anlagenexternen Quellen zur Verfügung gestellt wird, und zu einem Teil um einen sogenannten Recyclestrom, der in dem Verfahren selbst gewonnen wird, wie unten erläutert. Auch die anderen Spaltöfen 12 und 13 können mit entsprechenden Strömen beschickt werden. Unterschiedliche Ströme können auch in unterschiedliche Spaltöfen 11 bis 13 eingespeist werden, ein Strom kann auf mehrere Spaltöfen aufgeteilt werden oder mehrere Teilströme können zu einem Sammelstrom vereinigt werden, der beispielsweise als Strom A einem der Spaltöfen 11 bis 13 zugeführt wird.

Durch Dampfspalten in dem Dampfspaltprozess 10 wird ein Rohgasstrom B erhalten, der bisweilen bereits an dieser Stelle als Spaltgasstrom bezeichnet wird. Der Rohgasstrom B wird in einer Reihe nicht dargestellter Aufbereitungsstufen eines Aufbereitungsprozesses 20 aufbereitet, beispielsweise einem sogenannten Ölquench unterzogen, vorfraktioniert, verdichtet, weiter gekühlt und getrocknet.

Der entsprechend behandelte Strom B, das eigentliche Spaltgas C, wird anschließend einem Trennprozess 30 unterworfen. In diesem wird eine Anzahl von Fraktionen gewonnen, die hier, wie eingangs erläutert, entsprechend der Kohlenstoffanzahl der überwiegend enthaltenen Kohlenwasserstoffe bezeichnet werden. Der in der Figur 1A dargestellte Trennprozess 30 arbeitet nach dem Prinzip "Demethanizer First", ein Trennprozess nach dem Prinzip "Deethanizer First" ist in Figur 1B dargestellt.

In dem Trennprozess 30 wird aus dem Spaltgas C zunächst in einer ersten Trenneinheit 31 (dem sogenannten Demethanizer) eine C1- bzw. C1minus-Fraktion (mit dem Bezugszeichen C1 bezeichnet) gasförmig abgetrennt, die, falls nicht bereits zuvor entfernt, auch noch Wasserstoff enthalten kann. Sie wird typischerweise als Brenngas verwendet. Es verbleibt eine flüssige C2plus-Fraktion (Bezugszeichen C2+), die in eine zweite Trenneinheit 32 (den sogenannten Deethanizer) überführt wird.

In der zweiten Trenneinheit 32 wird aus der C2plus-Fraktion eine C2-Fraktion (Bezugszeichen C2) gasförmig abgetrennt und beispielsweise einem Hydrotreatmentprozess 41 unterzogen, um enthaltenes Acetylen zu Ethylen umzusetzen. Anschließend wird die C2-Fraktion in einer C2-Trenneinheit 35 in Ethylen (Bezugszeichen C2H4) und Ethan (Bezugszeichen C2H6) aufgetrennt. Letzteres kann als Recyclestrom D in einem oder mehreren Spaltöfen 11 bis 13 erneut dem Dampfspaltprozess 10 unterworfen werden. Im dargestellten Beispiel werden die Recycleströme D und E zu dem Strom A zugegeben. Die Recycleströme D und E und der Strom A können auch in unterschiedliche Spaltöfen 11 bis 13 geführt werden.

In der zweiten Trenneinheit 32 verbleibt eine flüssige C3plus-Fraktion (Bezugszeichen C3+), die in eine dritte Trenneinheit 33 (den sogenannten Depropanizer) überführt wird. In der dritten Trenneinheit 33 wird aus der C3plus-Fraktion eine C3-Fraktion (Bezugszeichen C3) abgetrennt und einem weiteren Hydrotreatmentprozess 42 unterzogen, um in der C3-Fraktion enthaltenes Propylen zu Propen umzusetzen.

Anschließend wird die C3-Fraktion in einer C3-Trenneinheit 36 in Propen (Bezugszeichen C3H6) und Propan (Bezugszeichen C3H8) aufgetrennt. Letzteres kann als Recyclestrom E in einem oder mehreren Spaltöfen 11 bis 13, separat oder mit anderen Strömen, erneut dem Dampfspaltprozess 10 unterworfen werden.

In der dritten Trenneinheit 33 verbleibt entsprechend eine flüssige C4plus-Fraktion (Bezugszeichen C4+), die in eine vierte Trenneinheit 34 (den sogenannten Debutanizer) überführt wird. In der vierten Trenneinheit 34 wird aus der C4plus-Fraktion eine C4-Fraktion (Bezugszeichen C4) abgetrennt. Es verbleibt eine flüssige C5plus-Fraktion (Bezugszeichen C5+).

Es versteht sich, dass sämtliche der dargestellten Fraktionen auch geeigneten Nachbehandlungsschritten unterworfen werden können. Beispielsweise kann aus der C4-Fraktion, wie auch unten dargestellt, 1,3-Butadien abgetrennt werden. Es können ferner zusätzliche Recycleströme verwendet werden, die analog zu den Recycleströmen D und E dem Dampfspaltprozess 10 unterworfen werden können.

In Figur 1B ist der Ablauf eines alternativen Verfahrens zur Erzeugung von Kohlenwasserstoffen durch Dampfspalten gemäß dem Stand der Technik in Form eines schematischen Ablaufplans dargestellt. Auch hier ist Kern des Verfahrens ein Dampfspaltprozess 10, der unter Verwendung eines oder mehrerer Spaltöfen 11 bis 13 ablaufen kann. Im Gegensatz zu dem in Figur 1A veranschaulichten Verfahren wird hier das Spaltgas C einem alternativen Trennprozess 30 nach dem Prinzip "Deethanizer First" unterworfen.

In dem Trennprozess 30 wird dabei aus dem Spaltgas C zunächst in einer ersten Trenneinheit 37 eine C2minus-Fraktion (Bezugszeichen C2-) gasförmig abgetrennt, die überwiegend Methan, Ethan, Ethylen und Acetylen und, falls nicht bereits zuvor entfernt, auch noch Wasserstoff enthalten kann. Die C2minus-Fraktion wird insgesamt einem Hydrotreatmentprozess 43 unterzogen, um Acetylen zu Ethylen umzusetzen. Anschließend wird aus der C2minus-Fraktion in einer C2minus-Trenneinheit 38 eine C1-Fraktion abgetrennt und wie oben weiter verwendet. Es verbleibt eine C2-Fraktion, die in einer C2-Trenneinheit 35 wie oben in Ethylen und Ethan aufgetrennt wird. Letzteres kann auch hier als Recyclestrom D in einem oder mehreren Spaltöfen 11 bis 13 erneut dem Dampfspaltprozess 10 unterworfen werden. In der ersten Trenneinheit 37 verbleibt eine flüssige C3plus-Fraktion, die in den Trenneinheiten 33 bis 36 und der Hydrotreatmenteinheit 42, wie zu Figur 1 erläutert, behandelt wird.

Dem Fachmann sind, beispielsweise aus dem eingangs erwähnten Artikel "Ethylene" in Ullmann's Encyclopedia of Industrial Chemistry, mehrere weitere Verfahrensalternativen bekannt, die sich insbesondere in der Aufbereitung des Spaltgases C und/oder dem verwendeten Trennprozess unterscheiden.

Auch die C4-Fraktion kann in Teilen als entsprechender Recyclestrom in einem oder mehreren der Spaltöfen 11 bis 13 erneut dem Dampfspaltprozess 10 unterworfen werden. Insbesondere dann, wenn hierbei milde Spaltbedingungen verwendet werden, können in der C4-Fraktion enthaltene verzweigte C4-Verbindungen (iso-C4-Verbindungen) jedoch in geringerem Umfang als n-C4-Verbindungen umgesetzt werden und finden sich daher zu einem großen Teil erneut im Spaltgasstrom C wieder. Die iso-C4-Verbindungen werden daher mehrfach durch eine entsprechende Anlage zirkuliert. Die Folge einer derartigen milden Dampfspaltung ist damit eine deutliche Mengenerhöhung einiger Produktfraktionen, hier der iso-C4-Verbindungen, und eine damit verbundene Reduktion der Konzentration enthaltener Wertprodukte, hier beispielsweise von 1,3-Butadien, durch Verdünnungseffekte. Die Gewinnung der Wertprodukte wird dadurch erschwert bzw. kostspieliger. Die iso-C4-Verbindungen tragen, mit anderen Worten, strukturbedingt praktisch nicht zur 1,3-Butadienbildung bei. Die Bildung einer relativ hohen Menge weitgehend wertlosen Methans ist unvermeidbar, insbesondere dann, wenn die iso-C4-Verbindungen bis zur vollständigen Umsetzung recycelt werden.

Werden also C4-Fraktionen mit iso-C4-Verbindungen, gleich welcher Herkunft, unter milden oder sehr milden Bedingungen gespalten, resultieren hieraus wiederum C4-Produktfraktionen relativ hoher Mengenströme bei gleichzeitig niedriger 1,3-Butadienkonzentration.

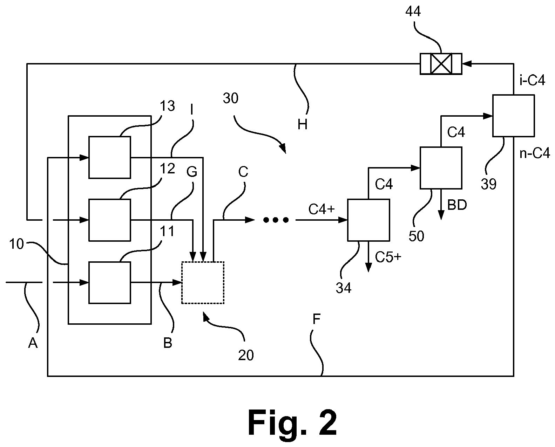

In Figur 2 ist der Ablauf eines Verfahrens zur Erzeugung von Kohlenwasserstoffen durch Dampfspalten gemäß einer Ausführungsform der Erfindung in Form eines schematischen Ablaufplans dargestellt. Auch hier ist Kern des Verfahrens ein Dampfspaltprozess 10, der unter Verwendung von Spaltöfen 11 bis 13 ablaufen kann. Zur Veranschaulichung der universellen Einsetzbarkeit des hier dargestellten Verfahrens ist die Gewinnung einer C4plus-Fraktion aus dem Spaltgas C nicht dargestellt, diese kann jedoch wie in den Figuren 1A und 1B oder auf jede andere in der Fachwelt bekannte Weise ablaufen. Die C4plus-Fraktion wird in dem hier dargestellten Beispiel einer Trenneinheit 34 zugeführt, die wie oben beschrieben arbeitet. Falls in einem Dampfspaltverfahren jedoch keine oder nur wenige C5plus-Kohlenwasserstoffe gebildet werden, könnte auf die Verwendung dieser Trenneinheit 34 auch verzichtet werden. Eine C4-Fraktion kann jedoch auch von anlagenextern, z.B. aus einer Raffinerie, bereitgestellt werden.

Eine beispielsweise aus der Trenneinheit 34 erhaltene C4-Fraktion kann einer 1,3-Butadiengewinnungseinheit 50 zugeführt werden, in der 1,3-Butadien, hier mit BD bezeichnet, extrahiert wird. 1,3-Butadien stellt hier eines der erwünschten Wertprodukte dar, die verbleibenden Anteile der C4-Fraktion C4 sind zu einem überwiegenden Anteil von geringerem wirtschaftlichem Wert und "verdünnen" das erwünschte 1,3-Butadien, was dessen Extraktion erschwert.

Die Erfindung sieht gemäß der dargestellten Ausführungsform vor, in einer Trenneinheit 39 iso-C4- und n-C4-Verbindungen (hier mit i-C4 und n-C4 bezeichnet), also verzweigte und unverzweigte C4-Verbindungen, voneinander zu trennen und entsprechende Teilströme zu gewinnen. Der Teilstrom, der überwiegend die iso-C4-Verbindungen enthält, wird hier als "erster Teilstrom" bezeichnet. Dieser kann als Recyclestrom H zurückgeführt und entweder erneut dem Dampfspaltprozess 10 oder einem separat zu dem Dampfspaltprozess 10 implementierten weiteren Dampfspaltprozess unterworfen werden. Vorzugsweise wird der erste Teilstrom mit den iso-C4-Verbindungen dabei scharfen Spaltbedingungen unterworfen, wofür hier der Spaltofen 12 eingerichtet sei. Zuvor kann eine Hydrierung von iso-Buten vorgenommen werden, wie mit Block 44 veranschaulicht. Ein dem Spaltofen 12 entnommener Strom G kann beispielsweise zu dem Spaltgas C zugegeben werden, ggf. nachdem dieser zuvor ebenfalls dem Aufbereitungsprozess 20 unterworfen wurde.

Der Teilstrom, der überwiegend die n-C4-Verbindungen enthält, kann als Recyclestrom F zurückgeführt und auch hier entweder erneut dem Dampfspaltverfahren 10 oder auch einem separat zu dem Dampfspaltprozess 10 implementierten weiteren Dampfspaltprozess unterworfen werden. Vorzugsweise werden die n-C4-Verbindungen dabei milden bis sehr milden Spaltbedingungen unterworfen, wofür hier der Spaltofen 13 eingerichtet sei. Ein dem Spaltofen 13 entnommener Strom I kann beispielsweise zu dem Spaltgas C zugegeben werden, ggf. nachdem dieser zuvor dem Aufbereitungsprozess 20 unterworfen wurde.

Wenngleich nicht dargestellt, versteht sich, dass den Spaltöfen 11 bis 13 noch weitere Recycleströme oder Frischeinsätze zugeführt werden können.

## Patentansprüche

1. Verfahren zur Erzeugung von Kohlenwasserstoffprodukten, das umfasst:
a) Bereitstellen eines Kohlenwasserstoffstroms (C4), der überwiegend verzweigte und unverzweigte Kohlenwasserstoffe mit jeweils vier Kohlenstoffatomen aufweist,
**gekennzeichnet durch**
b) Gewinnen eines ersten und eines zweiten Teilstroms (i-C4, n-C4) aus dem Kohlenwasserstoffstrom (C4), wobei der erste Teilstrom (i-C4) überwiegend verzweigte Kohlenwasserstoffe mit vier Kohlenstoffatomen und der zweite Teilstrom (n-C4) überwiegend unverzweigte Kohlenwasserstoffe mit vier Kohlenstoffatomen aufweist, und
c) Dampfspalten zumindest eines Teils des ersten Teilstroms (i-C4) oder eines hiervon abgeleiteten Stroms bei einer ersten, höheren Spaltschärfe und zumindest eines Teils des zweiten Teilstroms (n-C4) oder eines hiervon abgeleiteten Stroms bei einer zweiten, geringeren Spaltschärfe, wobei die erste, höhere Spaltschärfe in einer Umsetzung von iso-Butan in dem ersten Teilstrom von mehr als 91% und bis zu 99% und die zweite, geringere Spaltschärfe in einer Umsetzung von n-Butan in dem zweiten Teilstrom von weniger als 92% und mehr als 50% resultiert.

2. Verfahren nach Anspruch 1, bei dem der erste Teilstrom (i-C4) vor dem Dampfspalten bei der ersten, höheren Spaltschärfe zumindest teilweise einem Hydrierverfahren unterzogen wird.

3. Verfahren nach Anspruch 1 oder 2, bei dem der gemäß a) bereitgestellte Kohlenwasserstoffstrom (C4) zumindest teilweise aus zumindest einem durch Dampfspalten gemäß c) erhaltenen Spaltgas (C) erzeugt wird.

4. Verfahren nach einem der vorstehenden Ansprüche, bei dem der gemäß a) bereitgestellte Kohlenwasserstoffstrom (C4) zumindest teilweise aus einem Spaltgas (C) erzeugt wird, das durch Dampfspalten eines Frischeinsatzes (A), insbesondere von einer oder mehreren Erdölfraktionen, Erdgaskomponenten mit zwei bis vier Kohlenstoffatomen und/oder Erdgaskondensaten, gebildet wird.

5. Verfahren nach einem der vorstehenden Ansprüche, bei dem der gemäß a) bereitgestellte Kohlenwasserstoffstrom (C4) zumindest teilweise aus einem ungespaltenen Frischeinsatz (A), insbesondere aus einer oder mehreren Erdölfraktionen, Erdgas Erdgaskomponenten mit zwei bis vier Kohlenstoffatomen und/oder Erdgaskondensaten, gebildet wird.

6. Verfahren nach einem der vorstehenden Ansprüche, bei dem die erste, höhere Spaltschärfe in einer Umsetzung von iso-Butan in dem ersten Teilstrom von mehr als 92%, 93% oder 94% und die zweite, geringere Spaltschärfe in einer Umsetzung von n-Butan in dem zweiten Teilstrom von weniger als 90%, 88%, 86%, 84%, 82%, 80%, 78%, 76%, 74%, 72%, 70% oder 65% und/oder mehr als 60% resultiert.

7. Verfahren nach einem der vorstehenden Ansprüche, bei dem die erste, höhere Spaltschärfe um 1 bis 30%, insbesondere um 2 bis 20%, höher ist als die zweite, geringere Spaltschärfe.

8. Verfahren nach einem der vorstehenden Ansprüche, bei dem das Dampfspalten bei der ersten, höheren Spaltschärfe unter Verwendung einer Dampfmenge von 0,4 kg/kg, insbesondere von 0,2 bis 0,7 kg/kg, und das Dampfspalten bei der zweiten, geringeren Spaltschärfe unter Verwendung einer Dampfmenge von 0,4 kg/kg, insbesondere von 0,2 bis 0,7 kg/kg, durchgeführt wird.

9. Verfahren nach einem der vorstehenden Ansprüche, bei dem das Dampfspalten bei der ersten, höheren Spaltschärfe und/oder bei der zweiten, geringeren Spaltschärfe jeweils in wenigstens einem Spaltofen (12, 13) durchgeführt wird, dem zumindest ein weiterer Ofeneinsatz (A) in Form wenigstens eines Recyclestroms und/oder wenigstens eines Frischeinsatzes zugeführt wird.

10. Verfahren nach einem der vorstehenden Ansprüche, bei dem 1,3-Butadien (BD) vor dem Gewinnen des ersten und des zweiten Teilstroms (i-C4, n-C4) gemäß b) aus dem Kohlenwasserstoffstrom (C4) abgetrennt wird.

11. Verfahren nach Anspruch 10, bei dem in dem Kohlenwasserstoffstrom (C4) enthaltenes Isobuten nach der Abtrennung des 1,3-Butadiens (BD) zumindest teilweise zu einem tert-Butylether umgesetzt und der tert-Butylether ebenfalls aus dem Kohlenwasserstoffstrom (C4) abgetrennt wird.

12. Verfahren nach Anspruch 11, bei dem in dem Kohlenwasserstoffstrom (C4) enthaltenes 1-Buten zumindest teilweise zu 2-Buten hydroisomerisiert wird.

## Claims

1. Process for generating hydrocarbon products which comprises:
a) providing a hydrocarbon stream (C4) comprising predominantly branched and unbranched hydrocarbons each having four carbon atoms,
**characterized by**
b) obtaining a first and a second substream (i-C4, n-C4) from the hydrocarbon stream (C4), wherein the first substream (i-C4) comprises predominantly branched hydrocarbons having four carbon atoms and the second substream (n-C4) comprises predominantly unbranched hydrocarbons having four carbon atoms and
c) steamcracking at least a portion of the first substream (i-C4) or a stream derived therefrom at a first, higher cracking severity and at least a portion of the second substream (n-C4) or a stream derived therefrom at a second, lower cracking severity, wherein the first, higher cracking severity results in a conversion of isobutane in the first substream of more than 91% and up to 99% and the second, lower cracking severity results in a conversion of n-butane in the second substream of less than 92% and more than 50%.

2. Process according to Claim 1, in which the first substream (i-C4) before steamcracking at the first, higher cracking severity is at least partly subjected to a hydrogenation process.

3. Process according to Claim 1 or 2, in which the hydrocarbon stream (C4) provided according to a) is at least partly generated from at least one cracking gas (C) obtained by steamcracking according to c).

4. Process according to any of the preceding claims, in which the hydrocarbon stream (C4) provided according to a) is at least partly generated from a cracking gas (C) formed by steamcracking of a fresh input (A), in particular of one or more crude oil fractions, natural gas components having two to four carbon atoms and/or natural gas condensates.

5. Process according to any of the preceding claims, in which the hydrocarbon stream (C4) provided according to a) is at least partly formed from an uncracked fresh input (A), in particular from one or more crude oil fractions, natural gas natural gas components having two to four carbon atoms and/or natural gas condensates.

6. Process according to any of the preceding claims, in which the first, higher cracking severity results in a conversion of isobutane in the first substream of more than 92%, 93% or 94% and the second, lower cracking severity results in a conversion of n-butane in the second substream of less than 90%, 88%, 86%, 84%, 82%, 80%, 78%, 76%, 74%, 72%, 70% or 65% and/or more than 60%.

7. Process according to any of the preceding claims, in which the first, higher cracking severity is 1 to 30%, in particular 2 to 20%, higher than the second, lower cracking severity.

8. Process according to any of the preceding claims, in which the steamcracking at the first, higher cracking severity is performed using a steam quantity of 0.4 kg/kg, in particular of 0.2 to 0.7 kg/kg, and the steamcracking at the second, lower cracking severity is performed using a steam quantity of 0.4 kg/kg, in particular of 0.2 to 0.7 kg/kg.

9. Process according to any of the preceding claims, in which the steamcracking at the first, higher cracking severity and/or at the second, lower cracking severity is in each case performed in at least one cracking furnace (12, 13) which is supplied with at least one further furnace input (A) in the form of at least one recycling stream and/or at least one fresh input.

10. Process according to any of the preceding claims, in which 1,3-butadiene (BD) is removed from the hydrocarbon stream (C4) before the obtaining of the first and the second substreams (i-C4, n-C4) according to b).

11. Process according to Claim 10, in which after the removal of the 1,3-butadiene (BD) isobutene present in the hydrocarbon stream (C4) is at least partly converted into a tert-butyl ether and the tert-butyl ether is likewise removed from the hydrocarbon stream (C4).

12. Process according to Claim 11, in which 1-butene present in the hydrocarbon stream (C4) is at least partly hydroisomerized to afford 2-butene.

## Revendications

1. Procédé de génération de produits hydrocarbonés, qui comprend :
a) la préparation d'un courant d'hydrocarbures (C4), qui comprend principalement des hydrocarbures ramifiés et non ramifiés, contenant chacun quatre atomes de carbone,
**caractérisé par**
b) l'obtention d'un premier et d'un deuxième courant partiel (i-C4, n-C4) à partir du courant d'hydrocarbures (C4), le premier courant partiel (i-C4) comprenant principalement des hydrocarbures ramifiés de quatre atomes de carbone et le deuxième courant partiel (n-C4) comprenant principalement des hydrocarbures non ramifiés de quatre atomes de carbone, et
c) le vapocraquage d'au moins une partie du premier courant partiel (i-C4) ou d'un courant dérivé de celui-ci à une première sévérité plus élevée et d'au moins une partie du deuxième courant partiel (n-C4) ou d'un courant dérivé de celui-ci à une deuxième sévérité plus basse, la première sévérité plus élevée résultant en une conversion de l'isobutane dans le premier courant partiel de plus de 91 % et de jusqu'à 99 %, et la deuxième sévérité plus basse en une conversion du n-butane dans le deuxième courant partiel de moins de 92 % et de plus de 50 %.

2. Procédé selon la revendication 1, selon lequel le premier courant partiel (i-C4) est au moins partiellement soumis à un procédé d'hydrogénation avant le vapocraquage à la première sévérité plus élevée.

3. Procédé selon la revendication 1 ou 2, selon lequel le courant d'hydrocarbures (C4) préparé selon a) est généré au moins partiellement à partir d'au moins un gaz de craquage (C) obtenu par vapocraquage selon c).

4. Procédé selon l'une quelconque des revendications précédentes, selon lequel le courant d'hydrocarbures (C4) préparé selon a) est généré au moins partiellement à partir d'un gaz de craquage (C), qui est formé par vapocraquage d'une charge fraîche (A), notamment d'une ou de plusieurs fractions de pétrole, d'un ou de plusieurs composants de gaz naturel contenant deux à quatre atomes de carbone et/ou d'un ou de plusieurs condensats de gaz naturel.

5. Procédé selon l'une quelconque des revendications précédentes, selon lequel le courant d'hydrocarbures (C4) préparé selon a) est formé au moins partiellement à partir d'une charge fraîche (A) non craquée, notamment d'une ou de plusieurs fractions de pétrole, d'un ou de plusieurs composants de gaz naturel contenant deux à quatre atomes de carbone et/ou d'un ou de plusieurs condensats de gaz naturel.

6. Procédé selon l'une quelconque des revendications précédentes, selon lequel la première sévérité plus élevée résulte en une conversion de l'isobutane dans le premier courant partiel de plus de 92 %, 93 % ou 94 %, et la deuxième sévérité plus basse en une conversion du n-butane dans le deuxième courant partiel de moins de 90 %, 88 %, 86 %, 84 %, 82 %, 80 %, 78 %, 76 %, 74 %, 72 %, 70 % ou 65 % et/ou de plus de 60 %.

7. Procédé selon l'une quelconque des revendications précédentes, selon lequel la première sévérité plus élevée est supérieure de 1 à 30 %, notamment de 2 à 20 %, à la deuxième sévérité plus basse.

8. Procédé selon l'une quelconque des revendications précédentes, selon lequel le vapocraquage à la première sévérité plus élevée est réalisé en utilisant une quantité de vapeur de 0,4 kg/kg, notamment de 0,2 à 0,7 kg/kg, et le vapocraquage à la deuxième sévérité plus basse en utilisant une quantité de vapeur de 0,4 kg/kg, notamment de 0,2 à 0,7 kg/kg.

9. Procédé selon l'une quelconque des revendications précédentes, selon lequel le vapocraquage à la première sévérité plus élevée et/ou à la deuxième sévérité plus basse est réalisé à chaque fois dans au moins un four de craquage (12, 13), dans lequel au moins une charge de four supplémentaire (A) sous la forme d'au moins un courant de recyclage et/ou d'au moins une charge fraîche est introduite.

10. Procédé selon l'une quelconque des revendications précédentes, selon lequel du 1,3-butadiène (BD) est séparé du courant d'hydrocarbures (C4) avant l'obtention du premier et du deuxième courant partiel (i-C4, n-C4) selon b).

11. Procédé selon la revendication 10, selon lequel l'isobutène contenu dans le courant d'hydrocarbures (C4) est au moins partiellement transformé en un éther tert.-butylique après la séparation du 1,3-butadiène (BD), et l'éther tert.-butylique est également séparé du courant d'hydrocarbures (C4).

12. Procédé selon la revendication 11, selon lequel le 1-butène contenu dans le courant d'hydrocarbures (C4) est au moins partiellement hydroisomérisé en 2-butène.
